(19) Europäisches Patentamt
European Patent Office
Office européen des brevets

(11) Veröffentlichungsnummer: **0 593 098 A2**

(12) # EUROPÄISCHE PATENTANMELDUNG

(21) Anmeldenummer: **93120072.9**

(22) Anmeldetag: **08.09.90**

(51) Int. Cl.$^5$: **B01D 15/00**, A01N 1/02, A61L 2/00

Diese Anmeldung ist am 13 - 12 - 1993 als Teilanmeldung zu der unter INID-Kode 60 erwähnten Anmeldung eingereicht worden.

(30) Priorität: **13.09.89 DE 3930510**

(43) Veröffentlichungstag der Anmeldung: **20.04.94 Patentblatt 94/16**

(60) Veröffentlichungsnummer der früheren Anmeldung nach Art. 76 EPÜ: **0 491 757**

(84) Benannte Vertragsstaaten: **AT BE CH DE DK ES FR GB GR IT LI SE**

(71) Anmelder: **BLUTSPENDEDIENST DER LANDESVERBÄNDE DES DEUTSCHEN ROTEN KREUZES NIEDERSACHSEN, OLDENBURG UND BREMEN G.G.m.b.H.**
**Eldagsener Strasse 38**
**D-31830 Springe(DE)**

(72) Erfinder: **Mohr, Harald**
**Walter-Flex-Strasse 27**
**D-30117 Hannover(DE)**

(74) Vertreter: **Schupfner, Gerhard D.**
**Müller, Schupfner & Gauger,**
**Karlstrasse 5**
**D-21244 Buchholz (DE)**

(54) **Verfahren und Vorrichtung zur selektiven Entfernung von photodynamischen Substanzen aus Blut und Blutprodukten.**

(57) Die Erfindung betrifft ein Verfahren zur selektiven Entfernung von photodynamischen Substanzen aus Blut und Blutprodukten, wobei die Reinigung über Adsorptionsmittel erfolgt. Als besonders vorteilhaft hat sich hierfür eine Anordnung erwiesen, bei der das Blut bzw. die betreffende Blutfraktion über eine Trennsäule geleitet wird, die das Adsorptionsmittel enthält.

EP 0 593 098 A2

Die Erfindung betrifft ein Verfahren und eine Vorrichtung zur selektiven Entfernung von photodynamischen Substanzen aus Blut und Blutprodukten.

Es ist bekannt, daß photodynamische Substanzen in Verbindung mit sichtbarem Licht oder UV-Licht virusinaktivierend wirken können. Ursache hierfür ist die Affinität dieser Stoffe zu äußeren Virusstrukturen oder zur viralen Nukleinsäure. Beides trifft für Phenothiazinfarbstoffe zu. Sie reagieren mit den Membranstrukturen umhüllter Viren und schädigen diese unter Lichteinfluß irreversibel, wodurch das Virus seine Infektiosität verliert (vgl. Snipes, W. et al., 1979, Photochem. and Photobiol. 29, 785-790).

Photodynamische Substanzen interagieren aber auch mit viraler RNA oder DNA, insbesondere mit den Guaninresten dieser Nukleinsäuren. Nach Bildung eines Farbstoff-Nukleinsäure-Komplexes wird dieser durch Lichtenergie angeregt, so daß es zur Denaturierung der Nukleinsäure und letztlich zu Strangbrüchen kommt. Weiterhin bewirken Phenothiazinfarbstoffe die Umwandlung von molekularem Sauerstoff in Sauerstoffradikale, die hoch reaktiv sind und auf verschiedene Weise viruzid wirken können (vgl. Hiatt, C.W., 1972, in: Concepts in Radiation Cell Biology, pp. 57-89, Academic Press, New York; Oh Uigin et al., 1987, Nucl. Acid Res. 15, 7411-7427).

Phenothiazinfarbstoffe wie Methylenblau, Neutralrot und Toluidinblau spielen hierbei eine besondere Rolle, weil sie schon in Verbindung mit sichtbarem Licht eine R2eihe von Viren inaktivieren können, darunter auch solche, die sich in humanem Plasma befinden und keine Lipidhüllen besitzen.
Hinzu kommt, daß z.B. Methylenblau (MB) und Toluidinblau (TB) selbst therapeutische Anwendung finden, u.a. als Antidot be2i Kohlenmonoxidvergiftungen und in der Langzeittherapie psychotischer Erkrankungen. Hierbei kommen ohne bedeutsame Nebenwirkungen Mengen von MB bzw. TB zur Anwendung (1 - 2 mg/kg Körpergewicht), die weit höher sind als diejenigen, die zur Virusinaktivierung notwendig sind. Die geringen Toxizitäten von MB und TB werden auch durch tierexperimentelle Daten belegt.

Allerdings ist nicht in allen Fällen geklärt, wie sich die photodynamischen Farbstoffe im Organismus verhalten, so daß eine Entfernung des Farbstoffes aus dem Blut oder den Blutprodukten wichtig sein kann.

Aus Heinmets et al. (Inactivation of Viruses in Plasma by Photosensitized Oxidation, Research Report, Walter Reed Army Institute of Research, Walter Reed Army Medical Center, Washington D.C., 1955) ist bereits ein Adsorptionsmittel bekannt, mit dem man Toluidinblau aus Plasma entfernen kann. Hierbei handelt es sich um ein Ionentauscher-Harz (Dowex 50), das allerdings nur eine unspezifische Farbstoffbindung zuläßt, so daß u.U. auch Plasmaproteine gebunden werden. Ein entsprechend unspezifischer Kationentauscher wurde später noch von Chang und Weinstein (Photodynamic Inactivation of Herpesvirus Hominis by Methylene Blue, Proceedings of the Society for Experimental Biology and Medicine 148, 291-293, 1975) beschrieben.

Die Aufgabe der vorliegenden Erfindung ist es daher, ein Verfahren und eine Vorrichtung zur selektiven Entfernung von photodynamischen Substanzen aus Blut und Blutprodukten bereitzustellen.

Erfindungsgemäß erfolgt die Lösung der Aufgabe dadurch, daß man das Blut oder die Blutprodukte über Adsorptionsmittel leitet, wobei man Adsorptionsmittel auf Basis von Polystyrol oder Polyacrylamid, insbesondere auf Basis von Polystyrol-Divinylbenzol oder Acrylester-Polymerisat, oder Silica-Gele oder Kieselgele verwendet.

Nach einer bevorzugten Ausgestaltung der Erfindung wird das Adsorptionsmittel mit dem Blut oder den Blutprodukten vermischt und anschließend wieder abgetrennt.

Besondere Vorteile erzielt man auch damit, daß man das Blut oder die Blutprodukte über eine Trennsäule leitet, die das Adsorptionsmittel enthält.

Zur Durchführung des Verfahrens eignet sich insbesondere eine Vorrichtung, bestehend aus mindestens einem Behältnis, dem eine Trennsäule nachgeschaltet ist, die das Adsorptionsmittel enthält. Eine derartige Vorrichtung hat den Vorteil, daß hierdurch ein System entsteht, mit dem man auf einfache Weise und unter geringem Kontaminationsrisiko beispielsweise virusinaktivierte Plasmaprotein9präparate, auch von einzelnen Donoren stammend, herstellen kann.

Einen besonderen Vorteil bietet hierbei die Verwendung von Blutbeuteln, die eine einfache Handhabung ermöglichen.

Überraschenderweise wurde gefunden, daß Methylenblau und andere Phenothiazinfarbstoffe sehr stark an verschiedene, kommerziell erhältliche Trenngele binden, die keinerlei oder nur geringe Proteinbindung besitzen. Derartige Adsorbentien sind also für die nachträgliche Entfernung der photodynamischen Farbstoffe besonders geeignet, weil sie diese Stoffe adsorbieren, jedoch zu keiner Veränderung des Blutes bzw. der Blutprodukte führen.

Geeignete Adsorbentien sind Silica-Gele bzw. Kieselgele mit Porenweiten, die so gering sind, daß Plasmaproteine nicht in die Gelmatrix eindringen können, wohl aber die niedermolekularen Farbstoffmoleküle, die dann infolge ionischer, elektrostatischer und hydrophober Wechselwirkungen gebunden werden.

Geeignet sind ferner Adsorbentien auf Basis von Polystyrol-Divinylbenzol bzw. Acrylester-Polymerisat. Sie werden gleichfalls mit geeigneten Porengrößen hergestellt und dienen hauptsächlich dazu, um aus wässrigen Lösungen nichtpolare Substanzen bzw. oberflächenaktive Stoffe, z. B. Detergentien, zu entfernen. Sie sind nicht oder nur schwach polar.

Beispiele für einige kommerziell erhältliche Adsorbentien:

| Adsorbens | Hersteller bzw. Lieferant |
|---|---|
| Daltosil 75 | Serva, Heidelberg |
| Si 100-Polyol RP 18 | Serva, Heidelberg |
| Kieselgel 40 | Merck, Darmstadt |
| Nucleosil 50 A | Machery & Nagel, Düren |
| Nucleosil 100 A | Machery & Nagel, Düren |
| Vydac SC-201 RP | Machery & Nagel, Düren |
| CPG 40 | Pierce Europe (Gebr. Faust GmbH), 5000 Köln |
| Bio Beads | Bio Rad, München |
| Amberlite Adsorberharze | Rohm & Haas, Frankfurt |

Als Blut bzw. Blutprodukte kommen unter anderem in Frage:

- Vollblut
- Erythrozytenkonzentrate
- Thrombozytenkonzentrate
- Plasma
- Serum
- Kryopräzipitat
- Konzentrate von Gerinnungsfaktoren
- Inhibitoren
- Fibronectin
- Albumin.

Hieraus lassen sich Phenothiazine der folgenden Strukturformel selektiv entfernen:

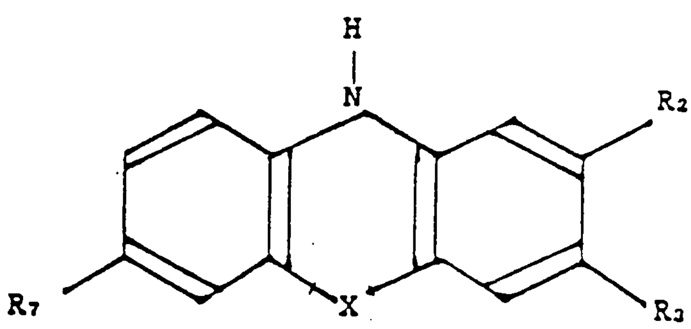

| | X | $R_2$ | $R_3$ | $R_7$ |
|---|---|---|---|---|
| Neutralrot | N | $CH_3$ | $NH_2$ | $N(CH_3)_2$ |
| Toluidinblau | S | $CH_3$ | $NH_2$ | $N(CH_3)_2$ |
| Methylenblau | S | H | $N(CH_3)_2$ | $N(CH_3)_2$ |
| Phenothiazin | S | H | H | H |

Anhand der folgenden Beispiele wird das erfindungsgemäße Verfahren näher beschrieben.

**Beispiel 1:**

Frischplasma wurde mit Methylenblau (10 µM) versetzt. 5 ml Aliquots wurden mit verschiedenen Mengen an Daltosil (Porenweite 75 A) bzw. Bio Beads SM 16 (Porenweite 144 A) versetzt und für 30 Minuten vermischt. Dann ließ man das Gel absitzen. Im Plasma wurden die Faktor VIII- bzw. Faktor V-Gehalte, die Extinktionen bei 660 nm sowie von einzelnen Proben die Proteingehalte gemessen.

**Tabelle 1:**

Extraktion von Methylenblau im Beutel

| | E (660 nm) | Protein (mg/ml) | Faktor VIII (U/ml) | Faktor V (U/ml) |
|---|---|---|---|---|
| Frischplasma | 0,909 | 66,8 | 1,10 | 1,20 |
| Frischplasma+MB | 1,450 | 65,6 | 0,42 | 0,96 |
| Daltosil 75 | | | | |
| 50 mg | 0,576 | -- | 0,60 | 1,05 |
| 100 mg | 0,571 | -- | 1,10 | 1,10 |
| 250 mg | 0,491 | -- | 1,10 | 1,20 |
| 500 mg | 0,477 | 66,8 | 1,25 | 1,20 |
| Bio Beads SM 16 | | | | |
| 50 mg | 0,666 | -- | 0,82 | 1,05 |
| 100 mg | 0,571 | -- | 1,05 | 1,10 |
| 250 mg | 0,571 | -- | 1,05 | 1,10 |
| 500 mg | 0,530 | 72,5 | 0,80 | 1,15 |

Aus den Extinktionswerten ist ersichtlich, daß offenbar neben dem Farbstoff noch weitere Substanzen aus dem Plasma extrahiert werden. Es handelt sich hierbei jedoch nicht um Plasmaproteine. Die Extinktionswerte des Plasmas, das mit 100 bis 250 mg Adsorbens pro 5 ml, d.h. mit 2 - 5 Gewichtsprozenten (% w/v) behandelt worden war, unterscheiden sich kaum von denen, bei welchen mit 10 % w/v Adsorbens extrahiert worden war. Es ergibt sich somit, daß bei einer MB-Konzentration von 10 µM in beiden Fällen 2 bis 5 % w/v Adsorbens ausreichen, um bei batchweisem Betrieb den Farbstoff aus dem Plasma zu entfernen. Entsprechend weniger Adsorbens wird benötigt, wenn die Einsatzkonzentration des Farbstoffs niedriger ist.

**Beispiel 2:**

In einem weiteren Versuch wurde anstatt Blutplasma eine 5%ige humane Serumalbuminlösung (5 % HSA) eingesetzt. Die MB-Konzentration betrug wiederum 10 µM 5 ml Aliquots wurden im Batch mit jeweils 100 mg, d.h. 2 % w/v folgender Adsorbentien für verschiedene Zeiten extrahiert: Daltosil (Porenweite 75 A), Kieselgel (Porenweite 40 A) sowie Bio Beads SM 16 (Porenweite 144 A).

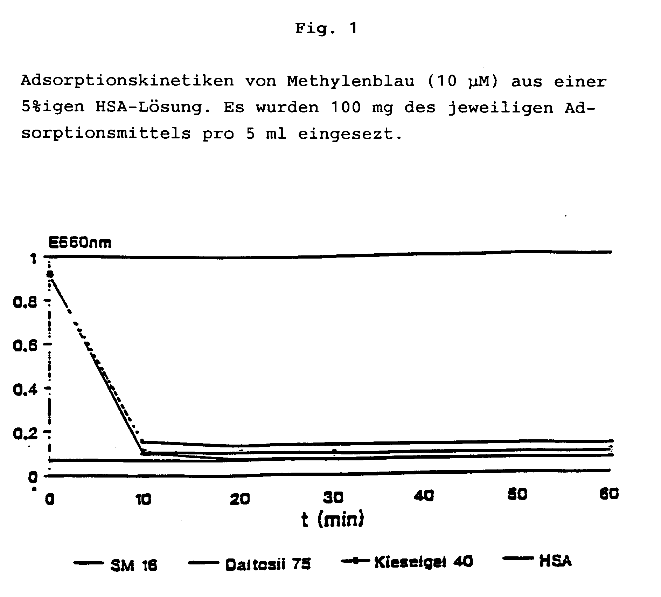

Wie die **Fig. 1** zeigt, geht in allen drei Fällen die Extinktion bei 660 nm innerhalb von 20 - 30 Minuten auf einen konstanten Wert zurück, d.h. diese Zeit ist ausreichend, um das Photooxidans bei batchweisem

Betrieb aus einer Plasmaproteinlösung zu entfernen. Wie die **Fig. 1** auch zeigt, sind Bio Beads SM 16 und Kieselgel 40 im gegebenen Fall offenbar etwas bessere Adsorptionsmittel als Daltosil mit 75 A Porenweite.

Zur Durchführung des erfindungsgemäßen Verfahrens eignet sich insbesondere eine Vorrichtung, bestehend aus mindestens einem Behältnis, dem eine Trennsäule nachgeschaltet ist, die das Adsorptionsmittel enthält. In einer bevorzugten Ausgestaltung der Erfindung dient als Behältnis ein Blutbeutel, der beispielweise Blut oder Plasmaproteine enthält. Von diesem Blutbeutel aus wird beispielsweise eine Plasmaproteinlösung über eine Trennsäule geleitet, in der sich das Adsorptionsmittel befindet, das die Farbstoffe adsorbiert. Die gereinigte Plasmaproteinlösung kann anschließend in einem zweiten Blutbeutel aufgefangen werden.

Das folgende Versuchsbeispiel zeigt das Ergebnis einer solchen Farbstofftrennung.

**Beispiel 3:**

Zur Farbstoffentfernung wurden 250 ml 5%iger Albuminlösung mit verschiedenen Geschwindigkeiten durch eine Trennsäule geleitet, die 5 ml Kieselgel (Porenweite 40 A) enthielt. 10 ml-Fraktionen wurden aufgefangen und ihre Extinktionswerte bei 660 nm gemessen.

Wie aus der **Tabelle 2** hervorgeht, ließ sich das Gesamtvolumen der Albuminlösung bei Durchflußgeschwindigkeiten von 5 bzw. 7,5 ml/min durch die Säule leiten, ohne daß restliches MB in den Durchlauffraktionen nachzuweisen war. Der Zeitaufwand, um aus 250 ml Lösung den Farbstoff zu entfernen, liegt demzufolge bei höchstens 30 bis 35 Minuten.

Das Ergebnis des Versuches zeigt, daß die Abtrennung des Photooxidans keine Probleme bereitet, zum anderen wird belegt, daß die oben skizzierte Herstellung farbstoffdepletierter Plasmaproteinpräparate einzelner Donoren möglich ist.

**Tabelle 2:**

Abtrennung von MB aus einer 5%igen Albuminlösung mittels Kieselgel 40
MB-Konzentration 1 μM

| Ausgangsmaterial + MB | Durchlaufgeschwindigkeit (ml/min) | |
|---|---|---|
| | 5 | 7,5 |
| Extinktion (660 nm):0,067 Fraktion Nr.: | Extinktion (660 nm) | |
| 1 | 0,002 | 0,001 |
| 3 | 0,000 | 0,001 |
| 5 | 0,000 | 0,002 |
| 7 | 0,002 | 0,003 |
| 9 | 0,001 | 0,001 |
| 11 | 0,000 | 0,001 |
| 13 | 0,000 | 0,001 |
| 14 | 0,002 | 0,001 |

## Patentansprüche

1. Verfahren zur selektiven Entfernung von photodynamischen Substanzen aus Blut und Blutprodukten, **dadurch gekennzeichnet,** daß man das Blut oder die Blutprodukte über Adsorptionsmittel leitet, wobei man Adsorptionsmittel auf Basis von Polystyrol oder Polyacrylamid, insbesondere auf Basis von Polystyrol-Divinylbenzol oder Acrylester-Polymerisat, oder Silica-Gele oder Kieselgele verwendet.

2. Verfahren nach Anspruch 1, **dadurch gekennzeichnet,** daß man das Adsorptionsmittel mit dem Blut oder den Blutprodukten vermischt und anschließend wieder abtrennt.

3. Verfahren nach Anspruch 1, **dadurch gekennzeichnet,** daß man das Blut oder die Blutprodukte über eine Trennsäule leitet, die das Adsorptionsmittel enthält.

4. Vorrichtung zur Durchführung des Verfahrens nach Anspruch 1, bestehend aus mindestens einem Behältnis, dem eine Trennsäule nachgeschaltet ist, die das Adsorptionsmittel enthält.

**5.** Vorrichtung nach Anspruch 4,
**dadurch gekennzeichnet,**
daß sich an die Trennsäule ein weiteres Behältnis anschließt, das zur Aufnahme des gereinigten Blutes bzw. der gereinigten Blutprodukte dient.

**6.** Vorrichtung nach Anspruch 4 oder 5,
**dadurch gekennzeichnet,**
daß als Behältnis ein Blutbeutel vorgesehen ist.

Fig. 1

Adsorptionskinetiken von Methylenblau (10 µM) aus einer 5%igen HSA-Lösung. Es wurden 100 mg des jeweiligen Adsorptionsmittels pro 5 ml eingesezt.